# EUROPÄISCHE PATENTANMELDUNG

(11) **EP 4 279 077 A2**
(43) Veröffentlichungstag der Anmeldung: **22.11.2023**
(21) Anmeldenummer: 23201690.7
(22) Anmeldetag: 26.01.2017
(51) Int. Cl.: A61K 31/721

(54) **IMMUNPROPHYLAXE BEI REZIDIVIERENDEN BAKTERIELLEN INFEKTIONEN**

(30) Priorität: 27.01.2016 DE 102016101448
(62) Teilanmeldung aus: 17702813.1
(71) Anmelder: Strathmann GmbH & Co. Kg, 22459 Hamburg (DE)
(72) Erfinder: Behnke, Bert, 22459 Hamburg (DE); Peschel, Claudia, 22459 Hamburg (DE)
(74) Vertreter: Hamm&Wittkopp Patentanwälte PartmbB

(57) **Zusammenfassung**

Die vorliegende Erfindung betrifft die Verwendung von Dextran als Impfstoff, insbesondere zur prophylaktischen Behandlung von rezidivierenden Infektionen bakterieller Herkunft. Außerdem betrifft die vorliegende Erfindung eine Impfstoffzusammensetzung, die Dextran als einzigen Impfstoff und ein Adjuvans, vorzugsweise eine Aluminiumverbindung, enthält sowie einen Arzneimittel-Kit zur Herstellung der Impfstoffzusammensetzung.

## Beschreibung

Die vorliegende Erfindung betrifft die Verwendung von Dextran zur prophylaktischen Behandlung von bakteriellen und viralen Infektionen sowie eine Impfstoffzusammensetzung, die Dextran als einzigen Impfstoff enthält.

Dextran ist ein Gemisch von Polysacchariden des α-1,6-Glucan-Typs. Dextrane dienen Hefen und Bakterien als Reservestoffe und bestehen ausschließlich aus Glucose-Einheiten. Die in der Medizin zum Einsatz kommenden Dextran-Fraktionen werden üblicherweise durch Hydrolyse natürlicher Dextrane und anschließender Fraktionierung der Polymermischung erhalten. Die natürlichen Dextrane stammen üblicherweise aus dem Bakterienstamm *Leuconostoc mesenteroides* NRRL B-512 oder dessen Unterstämme, z.B. B-512(F). Die medizinisch zum Einsatz kommenden Dextran-Fraktionen werden durch die Angabe der mittleren relativen Molekülmasse charakterisiert. Die für die Herstellung von Parenteralia zugelassenen Dextran-Fraktionen Dextran 40, 60 und 70 weisen eine mittlere relative Molekülmasse von etwa 40.000, 60.000 bzw. 70.000 auf.

Außer zur Herstellung von Parenteralia kommen Dextrane in Form von wässrigen Lösungen als Blutplasmaersatzstoffe zum Einsatz. In der Chirurgie werden niedrigmolekulare Dextrane als Thrombozytenaggregationshemmer eingesetzt. Darüber hinaus werden Dextrane als stabilisierender Zusatz bei der Gefriertrocknung eingesetzt. So ist z.B. Dextran 40 als Schutz- und Stabilisatorsubstanz in dem Lyophilisat enthalten, das zusammen mit einer wässrigen Aluminiumphosphat-Suspension unter dem Handelsnamen StroVac^{®} für die Herstellung einer Injektionssuspension zur Therapie und Prophylaxe rezidivierender Hamwegsinfekte bakterieller Herkunft vermarktet wird. Das Lyophilisat enthält neben Dextran 40 verschiedene Salze, Saccharose und als Impfstoff ein Wirkstoffkonzentrat inaktivierter Keime (*Escherichia coli, Morganella morganii, Proteus mirabilis, Klebsiella pneumoniae* und *Enterococcus faecalis*)*.*

Dextran ist darüber hinaus immunologisch wirksam. Die Verwendung von Dextran als immunologischer Wirkungsverstärker, d.h. als Adjuvans, um die Immunantwort auf einen Impfstoff zu verstärken, wurde beschrieben. Außerdem war der Einsatz von Dextran als Bestandteil eines konjugierten Impfstoffs, dessen immunologische Wirksamkeit vornehmlich auf das an Dextran konjugierte Antigen beruht, bekannt. Die Verwendung von Dextran als Adjuvans oder als Bestandteil eines konjugierten Impfstoffs gegen HIV wird in US 6,287,568 beschrieben.

US 2006/0019926 offenbart die Verwendung von Dextran zur Vorbeugung einer Mastitis. Der Jahreszyklus einer Milchkuh ist durch eine etwa 305 Tage andauernde Laktationsperiode und eine etwa 60 Tage andauernde Trockenstehperiode, in der die Milchdrüsen regenerieren, gekennzeichnet. Während der Einleitung der Trockenstehperiode sind die Milchdrüsen sehr infektionsanfällig, da sie in den ersten Tagen der Trockenstehperiode noch nicht mit einem Keratin-Pfropf zum Schutz vor Keimen verschlossen sind. US 2006/0019926 schlägt nun die Verabreichung einer Dextran enthaltenden Depotformulierung vor, die in den Euter injiziert wird und Dextran langsam freisetzt, was einen anhaltenden Stimulus für das Immunsystem bewirkt.

Die humorale Immunantwort beinhaltet drei Phasen: Aktivierungsphase, Differenzierungsphase und Effektorphase. In der Aktivierungsphase werden B-Lymphozyten (B-Zellen), welche mit ihrem B-Zell-Rezeptor, der membranständige Vorläufer des Antikörpers, an ein Antigen gebunden haben, je nach Antigen entweder direkt oder mithilfe einer T-Helferzelle aktiviert. In der Differenzierungsphase bildet die aktivierte B-Zelle B-Plasmazellen und B-Gedächtniszellen aus, wobei erstere Antikörper produzieren, die das Pathogen (Antigen) unschädlich machen, und letztere langlebig sind, um bei einem späteren Kontakt mit demselben Antigen für eine schnellere und wirksamere Immunantwort zu sorgen. In der Effektorphase kommt es zur Antigen-Antikörper-Reaktion unter Ausbildung eines Immunkomplexes, der durch Makrophagen abgebaut wird.

Neben der Bildung von B-Gedächtniszellen (und T-Gedächtniszellen) bei der primären Immunantwort wird für die Ausbildung des immunologischen Gedächtnisses (Immunisierung), d.h. für die Befähigung des Organismus zur sekundären Immunantwort, ein zweiter Mechanismus diskutiert, nämlich die Persistenz des Antigens im Organismus. Dieser zweite Mechanismus führt zu einem vergleichsweise kürzeren immunologischen Gedächtnis, das durch eine langsame Abgabe des Antigens aus dem Immunkomplex und einen damit einhergehenden dauernden Stimulus für das Immunsystem bewirkt wird.

Es wird allgemein angenommen, dass sich die Differenzierung von aktivierten B-Zellen zu B-Gedächtniszellen in den sekundären lymphatischen Organen unter Ausbildung von Keimzentren vollzieht, und zwar unter Beteiligung von aktivierten T-Helferzellen. Demzufolge besteht die allgemeine Annahme, dass T-Zellen unabhängige Antigene, d.h. Antigene, die in der Lage sind, B-Zellen direkt zu aktivieren, keine B-Gedächtniszellen erzeugen können. Aus diesem Grund werden T-Zellen unabhängige Antigene für eine Immunisierung generell als ungeeignet erachtet, da sie, wenn überhaupt, nur ein relativ kurzes immunologisches Gedächtnis in Form des Immunkomplexes ausbilden können.

US 6,287,568 erwähnt, dass Polysaccharide mikrobieller Herkunft und Polyvinylpyrrolidon T-Zellen unabhängige Antigene sind und dass nach Verabreichung der Polysaccharide α-1,6/α-1,3-Glucan B1355S, Levan bzw. α-1,6-Glucan (Dextran) an Mäusen und Menschen eine vollständige Immunantwort erst nach mehreren Wochen zu beobachten war. US 6,287,568 lehrt nunmehr, dass die T-Zellen-Unabhängigkeit der Polysaccharid-Antigene diese insbesondere für die Immunisierung gegen HIV, Aids-bedingten opportunistischen Infektionen sowie anderen Infektionen mit T-Zelldefiziten prädestiniert, und zwar entweder als Adjuvans in Kombination mit T-Zell abhängigen Antigenen, insbesondere Glycoproteinen, oder als Bestandteil eines Polysaccharid/Antigen-Konjugatimpfstoffs. Als geeignete Polysaccharide haben sich insbesondere Dextrane mit einem Molekulargewicht von ≥ 90 kDa herausgestellt.

Polysaccharide werden in der Immunologie als Adjuvantien eingesetzt, um die Immunantwort auf eine verabreichte Substanz unspezifisch zu steigern. Diese Adjuvantien bewirken die Ausschüttung von Cytokinen wie TNF-α und IL-1, die über eine Co-Stimulation die Antigen-spezifische T- und B-Zell-basierte Immunantwort verstärken. Daneben wird Chemotaxis, d.h. der Einfluss von Polysacchariden auf die Migration von Lymphozyten zu der Injektionsstelle, und eine damit einhergehende verstärkte Aufnahme löslicher Antigene als Wirkmechanismus diskutiert. Geeignete Polysaccharid-Adjuvantien werden in Expert. Rev. Vaccines 2011, 10(4), 523-537, genannt, z.B. Zymosan, Mannan, Muramyl-Dipeptid (MDP), Lipopolysaccharide bakteriellen Ursprungs (LPS) und α- bzw. β-Glucan.

Außerdem wurde beobachtet, dass natives Dextran N279, das aus *Leuconostoc mesenteroides* NRRL B512 isoliert wurde und im Wesentlichen aus α-1,6-Glucan besteht, eine B-Zellenimmunantwort in Mäusen induziert, die mit einer Entwicklung von Keimzentren einhergeht. Eine entsprechende Keimzentrumentwicklung nach Verabreichung des α-1,6/α-1,3-Glucans B1355S konnte nicht beobachtet werden (Proc. Natl. Acad. Sci. USA 1994, 91, 2502-2506).

Des Weiteren wurde beobachtet, dass nach einer primären Immunisierung von Mäusen mit T-Zellen unabhängigen und T-Zellen abhängigen Formen des Dextrans, nämlich einmal mit nativen Dextran B512 und einmal mit dem Konjugat aus diesem Dextran und Hühnerserum-Albumin, die Keimzentren-Bildung zwar im gleichen Ausmaß induziert wurde, die Immunisierung mit dem T-Zellen unabhängigen Antigen aber nicht zur Ausbildung von B-Gedächtniszellen führte. Andererseits führte die Immunisierung von Mäusen mit dem nativen Dextran B512 in Anwesenheit eines Aluminium-Adjuvans zum verstärkten Auftreten von Arealen mit Dextran spezifischen B-Zellen, die vorzugsweise außerhalb der Keimzentren lokalisiert waren. Die Anwesenheit des Aluminium-Adjuvans führte zu einer anhaltenden Immunantwort (International Immunology 1998, 10(7), 851-859).

Es wurde nun überraschenderweise gefunden, dass Dextran als Impfstoff zur prophylaktischen Behandlung von bakteriellen und viralen Infektionen, insbesondere von bakteriellen Infektionen eingesetzt werden kann. Es konnte in einer randomisierten, doppel-blinden und Placebo-kontrollierten Parallelgruppenstudie zur Wirksamkeit und Verträglichkeit von StroVac^{®} bei Patienten mit rezidivierenden bakteriellen Harnwegsinfektionen festgestellt werden, dass das eigentliche Placebo, das sich von der StroVac^{®}-Injektionssuspension lediglich darin unterschied, dass es keine inaktivierten Keime enthielt, hinsichtlich der Wirksamkeit keine statistisch signifikanten Unterschiede zu der etablierten Impfung mit StroVac^{®} zeigt. Das Auftreten von bakteriellen Harnwegsinfektionen von im Mittel 5,4-mal in 12 Monaten vor Studieneinschluss konnte durch Verabreichung des Placebos auf 1,3 bakterielle Harnwegsinfektionen in 13,5 Monaten (StroVac^{®}: 5,5 auf 1,2 bakteriellen Harnwegsinfektionen in 13,5 Monaten) reduziert werden. Hingegen traten in der Placebo-Gruppe deutlich weniger Impfreaktionen als in der Verum-Gruppe auf. Somit konnte gezeigt werden, dass Dextran für die prophylaktische Behandlung von rezidivierenden bakteriellen Harnwegsinfektionen geeignet ist.

Dextran ist ein bakterielles Polysaccharid, das in Säugetieren nach parenteraler Verabreichung nur relativ langsam abgebaut wird. Deshalb kann vermutet werden, dass die immunologische Wirkung von Dextran auf die Ausbildung von relativ stabilen Dextran-Antidextran-Immunkomplexen beruht, die Dextran über einen relativ langen Zeitraum freisetzen und somit einen dauernden Stimulus für das Immunsystem verursachen. Andererseits kann auch nicht ausgeschlossen werden, dass die Impfung mit Dextran zur Entwicklung entsprechender B-Gedächtniszellen führt. Die Dextran spezifischen Antikörper der Klasse A werden in die Blasenschleimhaut sezerniert und sind dort in der Lage, mikrobielle Strukturen zu binden. Immunglobulin A wird von den B-Lymphozyten des MALT (Mucosaassoziiertes lymphatisches Gewebe) produziert, das für die Immunreaktion von Schleimhautoberflächen verantwortlich ist. Das gebildete Immunglobulin A verhindert die Anheftung von Bakterien und Viren an das Epithel und infolgedessen den Eintritt dieser Pathogene in subepitheliale Schichten. Es ist somit davon auszugehen, dass die Impfung mit Dextran eine Anheftung von Bakterien und Viren an das Schleimhaut-Epithel verhindert, so dass Dextran insbesondere zur prophylaktischen Behandlung von rezidivierenden bakteriellen und viralen Infektionen, wie z.B. bakteriellen Harnwegsinfektionen oder bakteriellen oder viralen Atemwegsinfektionen, geeignet ist.

Die vorliegende Erfindung betrifft des Weiteren eine Impfstoffzusammensetzung, die Dextran als einzigen Impfstoff und gegebenenfalls ein Adjuvans enthält. Vorzugsweise enthält die Impfstoffzusammensetzung ein Adjuvans, insbesondere eine Aluminiumverbindung. Als Beispiele für geeignete Aluminiumverbindungen können Aluminiumhydroxid und Aluminiumphosphat genannt werden. Der Wirkmechanismus von Aluminiumverbindungen als Adjuvantien in der Immunologie ist bekannt (SpringerPlus 2015, 4:181; Scientific Reports 2015, 5 Artikel-Nr. 13146). Aluminiumverbindungen, wie z.B. Aluminiumhydroxid und Aluminiumphosphat, sorgen aufgrund der durch die Aluminium-Injektion ausgelösten lokalen Entzündungsreaktion für eine erhöhte Aufnahme von Antigenen durch antigenpräsentierende Zellen. Die zum Injektionsort angelockten unreifen dendritischen Zellen nehmen lösliche Antigene auf, die mit der Aluminiumverbindung verabreicht wurden, d.h. erfindungsgemäß Dextran, und wandern in die Lymphknoten, wo sie die Aktivierung von B-Zellen einleiten. Aluminiumverbindungen sorgen somit für eine erhöhte Aufnahme von Antigenen durch antigenpräsentierende Zellen. In einer bevorzugten Ausführungsform der vorliegenden Erfindung ist die Impfstoffzusammensetzung eine Injektionssuspension, die insbesondere für die intramuskuläre Injektion hergerichtet ist.

Des Weiteren betrifft die vorliegende Erfindung einen Arzneimittel-Kit zur Herstellung einer Impfstoffzusammensetzung, die Dextran als einzigen Impfstoff und ein Adjuvans enthält, wobei eine erste Zusammensetzung des Kits Dextran enthält und eine zweite Zusammensetzung das Adjuvans. Vorzugsweise liegt die erste Zusammensetzung als Trockensubstanz und die zweite Zusammensetzung als wässrige Suspension vor. Die vorliegende Erfindung betrifft ebenfalls eine Impfstoffzusammensetzung, die als einzigen Impfstoff Dextran und gegebenenfalls ein Adjuvans enthält, sowie einen Arzneimittel-Kit, das Dextran in einer ersten Zusammensetzung und das Adjuvans in einer zweiten Zusammensetzung enthält, zur prophylaktischen Behandlung von bakteriellen und viralen Infektionen.

Die mittlere relative Molekülmasse des in der erfindungsgemäßen Verwendung eingesetzten Dextrans liegt üblicherweise im Bereich von 10.000-200.000, bevorzugt 20.000-120.000, mehr bevorzugt 30.000-100.000 und am meisten bevorzugt 40.000-80.000. Als Dextran zur Verwendung als Impfstoff können insbesondere die im Europäischen Arzneibuch (Ph. Eur. 8. Ausgabe, Grundwerk 2014) genannten Dextrane zur Herstellung von Parenteralia genannt werden, d.h. Dextran 40, 60 und 70 mit einer mittleren relativen Molekülmasse von etwa 40.000, etwa 60.000 bzw. etwa 70.000.

Die Dextran-Dosis für die Immunisierung liegt im Bereich von 15 mg-100 mg, vorzugsweise 20 mg-80 mg, und am meisten bevorzugt im Bereich von 30 mg-50 mg. Diese Dosis kann gegebenenfalls mit mehreren Injektionen verabreicht werden, z.B. mit 2 oder 3 Injektionen im Abstand von jeweils 1-2 Wochen. Mit der Immunisierung wird ein Schutz von etwa einem Jahr erreicht, so dass eine dauerhafte Immunisierung durch eine jährliche Verabreichung der erfindungsgemäßen Impfstoffzusammensetzung erzielt werden kann.

### Beispiele

### 1. Formulierungsbeispiel

### Beschreibung und Zusammensetzung des erfindungsgemäßen Arzneimittel-Kits:

Bis auf den Wegfall des Wirkstoffkonzentrats in dem StroVac^{®}-Placebo gleichen sich Verum und Placebo hinsichtlich der Zusammensetzung. Der Anteil des Wirkstoffkonzentrats wurde im Placebo durch PBS-0,01 %-Thiomersal-Puffer ersetzt.

### Eine Durchstechflasche mit Lyophilisat (Vial Trockensubstanz) enthält:

| | | |
|---|---|---|
| Saccharose | Ph.Eur.* | 12,9 - 14,375 mg |
| Dextran 40 | Ph.Eur.* | 12,9 - 14,375 mg |
| NaCl | Ph.Eur.* | 2,5 - 2,79 mg |
| Na₂HPO₄ x 2 H₂O | Ph.Eur.* | 0,49 - 0,54 mg |
| KH₂PO₄ | Ph.Eur.* | 0,30 - 0,33 mg |
| Thiomersal | Ph.Eur.* | 0,030 - 0,033 mg |

| | | |
|---|---|---|
| ** Ph. Eur. 8. Ausgabe* | | |

### Die eingesetzten Substanzen haben folgende Funktionen:

- Saccharose und Dextran 40 dienen als Schutz- und Stabilisatorsubstanzen während der Lyophilization.
- Dextran 40 ist der Impfstoff.
- Natriumchlorid, (Dinatriummonohydrogenphosphat-Dihydrat und Kaliumdihydrogenphosphat sind Bestandteile der phosphatgepufferten Salzlösung (PBS-Puffer).
- Thiomersal wird als Konservierungsmittel eingesetzt.

*Eine Ampulle mit Suspension (Basis-Suspension) enthält:*

| | | |
|---|---|---|
| Aluminiumphosphat | Company spec. | 1,0 mg |
| Wasser zur Injektion | Ph.Eur.* | 500,0 mg |

| | | |
|---|---|---|
| ** Ph. Eur. 8. Ausgabe* | | |

### 2. Beschreibung und Ergebnisse der klinischen Studie

### Herstellen der anwendungsbereiten Injektionssuspension (Impfsuspension):

Inhalt der Ampulle gründlich aufschütteln, mittels Spritze aufziehen und in Vial mit der Trockensubstanz injizieren. Gemisch erneut kräftig schütteln und anschließend wieder in die Spritze aufziehen. Vor der Injektion die Kanüle wechseln.

### Injektionen durchführen:

Es werden 3 Dosen Impfsuspension pro Behandlungszyklus benötigt. Es sind 3 intramuskuläre Injektionen vorzugsweise in den M. deltoideus im Abstand von jeweils 2 Wochen durchzuführen. Dabei soll langsam tief in den Muskel injiziert werden und beim Entfernen der Kanüle ein Rückstrom in die Subcutis mittels Tupferdruck vermieden werden.

### Anwendungsgebiet:

Zur Immunprophylaxe bei Patienten mit rezidivierenden bakteriellen Infektionen
- der Harnwege
- der Atemwege
- der Haut.

### Wirksamkeitsdaten:

Prospektive, multizentrische, randomisierte, doppel-blinde, Placebo-kontrollierte Parallelgruppenstudie zur Wirksamkeit und Verträglichkeit von StroVac^{®} bei Patienten mit rezidivierenden, symptomatischen, bakteriellen Harnwegsinfektionen. **Die erfindungsgemäße Suspension zur Immunprophylaxe wurde in der Studie als "Placebo" verwendet.**

### Einschlussdiagnose:

Mindestens einjährige Vorgeschichte rezidivierender akuter unkomplizierter symptomatischer bakterieller Harnwegsinfektionen. In die Studie wurden Patienten mit "informed consent" eingeschlossen, mit folgenden Einschlusskriterien:
1. Männer und Frauen im Alter von 18 bis 80 Jahren
2. Patienten mit einer mindestens einjährigen Vorgeschichte rezidivierender unkomplizierter symptomatischer bakterieller Harnwegsinfektionen
3. Patienten mit mindestens fünf dokumentierten Episoden von unkomplizierten symptomatischen bakteriellen Harnwegsinfektionen während eines Zeitraumes von zwölf Monaten vor Studieneinschluss.

Die wichtigsten Ausschlusskriterien waren:
1. Komplizierte Harnwegsinfektionen oder andere Erkrankungen der Harnwege wie nicht-bakterielle Zystitis oder mit Obstruktion einhergehende Erkrankungen/Anomalien wie Blasen- oder Nierensteine, Ureterstrikturen oder gutartige Prostatahyperplasie mit Harnverhalt (Bestätigung durch Ultraschall von Nieren und Blase und Bestimmung einer Restharnmenge von mehr als 50 ml).
2. Patienten mit überaktiver Blase (>8 Miktionen pro Tag bei unauffälligem Urinbefund)
3. Kontraindikationen für die Anwendung des Prüfpräparates:
   - akute Infektionen, mit Ausnahme von urogenitalen Infektionen
   - aktive Tuberkulose
   - schwerwiegende hämatopoetische Erkrankungen
   - schwerwiegende kardiovaskuläre und renale Erkrankungen
   - Erkrankungen des Immunsystems
   - Überempfindlichkeit auf StroVac^{®}
4. Störung der Immunität in Folge von Erkrankungen wie Diabetes mellitus mit instabilem Stoffwechselstatus oder Vorliegen manifester diabetischer Spätkomplikationen oder Leberinsuffizienz
5. Bösartige Erkrankungen, die weniger als 5 Jahre zurückliegen (ausgenommen Basaliome)
6. Strahlentherapie im Unterbauch (ohne zeitliche Begrenzung) oder Strahlentherapie eines anderen Körperbereichs innerhalb der letzten 5 Jahre vor Start der Studie oder geplant während der Studie
7. Einnahme von nicht-erlaubter Vorbehandlung.

### Ergebnisse zur Wirksamkeit für die erfindungsgemäße Suspension zur Immunprophylaxe:

Weder im primären Endpunkt noch in einem der sekundären Endpunkte gab es statistisch signifikante Unterschiede im Vergleich zu der etablierten Impfung StroVac^{®}, die außer den zum Placebo identischen Hilfsstoffen bakterielle Antigene typischer Harnwegsinfektionserreger enthält und zur Bildung spezifischer sIgA-Antikörper in der Blase führt. Die erfindungsgemäße Suspension zur Immunprophylaxe ("Placebo") zeigte eine große Wirkung bei Patienten mit rezidivierenden bakteriellen Harnwegsinfektionen:
1. Reduktion der bakteriellen Harnwegsinfektionen (HWI) von im Mittel 5,4 in 12 Monaten vor Studieneinschluss auf 1,3 HWI in 13,5 Monaten während der Studie (StroVac^{®} 5,5 auf 1,2 HWI).
2. In den 13,5 Monaten während der Studie hatten 44,7 % der Patienten unter "Placebo" überhaupt keine HWI mehr (StroVac^{®} 38,0 %).
3. Die Reduktion der HWIs erfolgte gleichmäßig sowohl im ersten als auch im zweiten Halbjahr nach Verabreichung der Studienmedikation: Median erste Halbjahr 0,6 HWI, 2.Halbjahr 0,5 HWI (StroVac^{®} 0,6 und 0,4).
4. Unklare HWIs wie monosymptomatische Bakteriurien, klinische Zystitiden ohne Bakteriennachweis und vom Patienten berichtete HWI ohne Arztbesuch traten unter "Placebo" ebenfalls nicht häufiger auf als unter StroVac^{®}. Auch wenn alle nachgewiesenen und möglichen HWIs zusammengezählt werden, ergibt sich für das "Placebo" eine Reduktion auf 2,2 HWI in den 12 Monaten nach Verabreichung der Studienmedikation. (StroVac^{®} 2,0 HWI).
5. Es gibt in beiden Gruppen eine gleichermaßen deutliche Verbesserung der symptombezogenen Lebensqualität (Symptom burden-score).
6. Die globale Wirksamkeitseinschätzung für das "Placebo" war für 71,8 % der Prüfärzte sehr gut oder gut (73,3% StroVac^{®}). Die Wirksamkeitseinschätzung der Patienten war für 69,7 % unter "Placebo" sehr gut oder gut (72,2 % StroVac^{®}).
7. Nonresponder am Studienende waren nach Einschätzung der Prüfärzte 23,4 % unter "Placebo" (StroVac^{®} 20,9 %). Nach Einschätzung der Patienten sprachen 26,1 % auf "Placebo" nicht an (22,5 % auf StroVac^{®}).
8. In der ad hoc analysierten Subgruppe der Patienten mit 7 und mehr HWIs in 12 Monaten vor Studieneinschluss zeigte sich erst eine signifikante Überlegenheit des Impfstoffs StroVac^{®} gegenüber dem "Placebo" und auch nur dann, wenn alle HWI (gesicherte und vermutete) dabei gezählt wurden.
9. 31 Patientinnen waren älter als 70 Jahre, davon 15 in der StroVac^{®}- und 16 in der "Placebo"-Gruppe. Unter Placebo hatten 56,3 % der Seniorinnen keine HWI in den 13,5 Monaten während der Studie (StroVac^{®} 26,7 %). Die mittlere Häufigkeit der HWI war 0,5 unter Placebo (1,3 unter StroVac^{®}). Auch hier lag kein statistisch signifikanter Unterschied vor.

### Verträglichkeitsdaten:

Als schwerwiegende Arzneimittelreaktionen waren in der Studie definiert Fieber >38,5 °C oder/und Schüttelfrost mit Auftreten innerhalb von 72 Stunden nach der Injektion. In der StroVac^{®} Gruppe lag die Inzidenz dieser SUAW, wie erwartet, bei 3,6 % der verabreichten Injektionen. Unter dem "Placebo" lag die Inzidenz diese SUAW nur bei 0,53 %.

Lokale Impfreaktionen mit einer oder mehreren Symptomen traten unter StroVac^{®} bei 28,3 % der Injektionen auf. Unter "Placebo" verursachten nur 3,4 % der Injektionen leichte lokale Reaktionen.

Hinsichtlich der globalen Verträglichkeitsbewertung war "Placebo" gegenüber StroVac^{®} signifikant überlegen.

## Patentansprüche

1. Dextran zur Verwendung als Impfstoff.

2. Dextran zur Verwendung gemäß Anspruch 1, wobei der Impfstoff für die prophylaktische Behandlung von bakteriellen und viralen Infektionen vorgesehen ist.

3. Dextran zur Verwendung gemäß Anspruch 2, wobei die bakteriellen und viralen Infektionen rezidivierende bakterielle und rezidivierende virale Infektionen sind.

4. Dextran zur Verwendung gemäß Anspruch 2 oder 3, wobei die Infektion eine bakterielle Harnwegsinfektion, bakterielle oder virale Atemwegsinfektion oder eine bakterielle Infektion der Haut ist.

5. Impfstoffzusammensetzung, die Dextran als einzigen Impfstoff und gegebenenfalls ein Adjuvans enthält.

6. Impfstoffzusammensetzung gemäß Anspruch 5, wobei die Zusammensetzung eine Aluminiumverbindung als Adjuvans enthält.

7. Impfstoffzusammensetzung gemäß Anspruch 6, wobei die Aluminiumverbindung Aluminiumhydroxid oder Aluminiumphosphat ist.

8. Impfstoffzusammensetzung gemäß einem der Ansprüche 5 bis 7 in Form einer Injektionssuspension.

9. Impfstoffzusammensetzung gemäß Anspruch 8, wobei die Injektionssuspension zur intramuskulären Injektion hergerichtet ist.

10. Arzneimittel-Kit zur Herstellung einer Impfstoffzusammensetzung gemäß einem der Ansprüche 5 bis 9, wobei eine erste Zusammensetzung Dextran enthält und eine zweite Zusammensetzung das Adjuvans.

11. Arzneimittel-Kit gemäß Anspruch 10, wobei die erste Zusammensetzung eine Trockensubstanz und die zweite Zusammensetzung eine wässrige Suspension ist.

12. Impfstoffzusammensetzung gemäß einem der Ansprüche 5 bis 9 oder Arzneimittel-Kit gemäß Anspruch 10 oder 11 zur prophylaktischen Behandlung von bakteriellen und viralen Infektionen.
